(19) Europäisches Patentamt
European Patent Office
Office européen des brevets

(11) Veröffentlichungsnummer: **0 050 857**
**A1**

(12) # EUROPÄISCHE PATENTANMELDUNG

(21) Anmeldenummer: **81108801.2**

(22) Anmeldetag: **23.10.81**

(51) Int. Cl.$^3$: **C 07 C 43/295**
C 07 C 121/75

(30) Priorität: **28.10.80 DE 3040487**

(43) Veröffentlichungstag der Anmeldung:
**05.05.82 Patentblatt 82/18**

(84) Benannte Vertragsstaaten:
**BE CH DE FR GB IT LI NL**

(71) Anmelder: **HOECHST AKTIENGESELLSCHAFT**
**Postfach 80 03 20**
**D-6230 Frankfurt/Main 80(DE)**

(72) Erfinder: **Welter, Wolfgang, Dr.**
**Gagernring 3**
**D-6233 Kelkheim (Taunus)(DE)**

(72) Erfinder: **Mildenberger, Hilmar, Dr.**
**Fasanenstrasse 24**
**D-6233 Kelkheim (Taunus)(DE)**

(54) **Phenoxyzimtalkohole, Verfahren zu ihrer Herstellung und ihre Verwendung als Zwischenprodukte zur Darstellung von Insektiziden.**

(57) Verbindungen der Formel

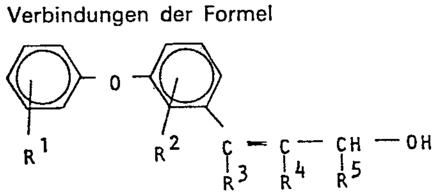

(1)

worin $R^1$ H, Halogen, Alkyl oder Alkoxy; $R^2$ H, Halogen, Alkyl oder $CF_3$; $R^3$ H, Cl, Br, Alkyl oder CN; $R^4$ H, Halogen, Alkyl, Phenyl oder Halogenphenyl und $R^5$ H, Alkyl, Alkinyl oder CN bedeuten, sind Ausgangsstoffe für die Herstellung wertvoller Insektizide von Pyrethroid-Typ.

EP 0 050 857 A1

Croydon Printing Company Ltd.

# Phenoxyzimtalkohole, Verfahren zu ihrer Herstellung und ihre Verwendung als Zwischenprodukte zur Darstellung von Insektiziden

Gegenstand der vorliegenden Erfindung sind Phenoxyzimtalkohole der Formel

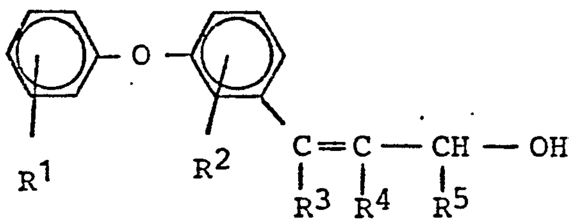

(I)

in welcher
$R^1$ Wasserstoff, Halogen, $(C_1-C_4)$Alkyl oder $(C_1-C_4)$Alkoxy,
$R^2$ Wasserstoff, Halogen, $(C_1-C_4)$Alkyl oder Trifluormethyl,
$R^3$ Wasserstoff, Cl, Br, $(C_1-C_4)$Alkyl oder Cyan
$R^4$ Wasserstoff, Halogen, $(C_1-C_4)$Alkyl, Phenyl oder Halogenphenyl,
$R^5$ Wasserstoff, $(C_1-C_4)$Alkyl, $(C_1-C_3)$Alkinyl oder Cyan
bedeuten.

Die allgemeine Formel (I) umfaßt auch die verschiedenen optischen und geometrischen Isomeren und deren Mischungen.

Man erhält die neuen Verbindungen der Formel I, indem man

(a) Verbindungen der Formel

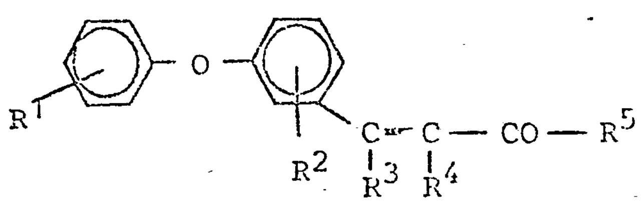

(II)

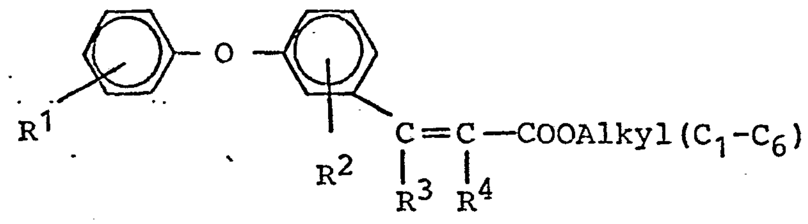

(III)

oder

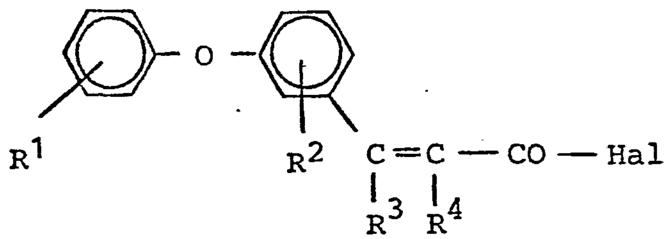

(IV)

reduziert, oder

(b) falls $R^5$ = CN,
Verbindungen der Formel (II) mit einem Alkali- oder Erdalkalicyanid in Gegenwart einer Säure bzw. mit Trimethylsilylcyanid behandelt, oder

(c) falls $R^5$ = Alkinyl,
Verbindungen der Formel (II) mit einer Grignard-Verbindung der Formel

$$R^6 - C \equiv C - MgHal \qquad (V)$$

worin $R^6$ H oder $CH_3$ ist, umsetzt.

Die Varianten (a) bis (c) zur Herstellung der erfindungsgemäßen Phenoxyzimtalkohole der allgemeinen Formel (I) werden bevorzugt unter Mitverwendung geeigneter Lösungs- oder Verdünnungsmittel durchgeführt.

Zur Durchführung der Variante (a) eignen sich vorzugsweise Ether, wie Diethylether, Tetrahydrofuran, Dioxan, sowie Kohlenwasserstoffe wie Toluol und Benzin. Bei Verwendung von Natriumborhydrid als Reduktionsmittel können zusätzlich Wasser, Alkohole wie Methanol, Ethanol, Nitrile

wie Acetonitril oder Propionitril verwendet werden. Zur Durchführung der Variante (b) eignen sich vorzugsweise Wasser, Alkohole wie Methanol, Ethanol oder Ether wie Diethylether, Tetrahydrofuran oder Nitrile wie Acetonitril. Für die Variante (c) eignen sich vorzugsweise Ether wie Diethylether, Tetrahydrofuran und Dioxan.

Als komplexe Metallhydride bei der Verfahrensvariante (a) seien vorzugsweise Lithiumaluminiumhydrid und Natriumborhydrid genannt.
Als Säuren bei der Verfahrensvariante (b) können anorganische Säuren, z.B. Salz- oder Schwefelsäure, oder organische Säuren, z.B. Essigsäure oder Ameisensäure verwendet werden.

Die Reaktionstemperatur kann bei allen Verfahren innerhalb eines größeren Bereichs variiert werden. Im allgemeinen arbeitet man bei -10°C bis 110°C;
bei Variante (a) vorzugsweise bei 0 bis 60°C,
bei Variante (b) vorzugsweise bei -5 bis 20°C,
bei Variante (c) vorzugsweise bei 0 bis 80°C.

Die Umsetzungen läßt man im allgemeinen bei Normaldruck ablaufen.

Zur Durchführung der Variante (a) setzt man die Reaktionspartner vorzugsweise in äquimolaren Mengen ein. Ein Überschuß der einen oder anderen Komponente bringt keinen Vorteil. Bei der Variante (b) wird das Cyanid - vorzugsweise NaCN oder KCN - vorteilhaft in 100 - 150 %igem Überschuß eingesetzt.

Die Aufarbeitung der Verfahrensprodukte kann auf übliche Weise erfolgen, indem man beispielsweise mit Wasser verdünnt, ansäuert, mit einem organischen Lösungsmittel extrahiert und nach dem Trocknen das Lösungsmittel abdestilliert.
Die neuen Verbindungen fallen in Form von Ölen an, die sich entweder destillieren lassen oder durch sogenanntes

"Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex oder der Siedepunkt.

Für die Herstellung der ebenfalls neuen Ausgangsstoffe der Formeln (II), (III) und (IV) stehen verschiedene Verfahren zur Verfügung. Die Ketone bzw. Aldehyde der Formel (II) erhält man z.B., indem man Verbindungen der Formel

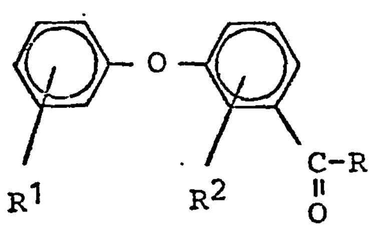

(VI)

worin R für Wasserstoff steht, mit Ketoverbindungen der Formel

$$R^4-CH_2-\underset{\underset{O}{\|}}{C}-R^5 \qquad \text{(VII)}$$

in Gegenwart einer Base und gegebenenfalls eines Verdünnungsmittels in an sich bekannter Weise umsetzt. Ein weiteres Verfahren besteht in der Umsetzung von Triphenylphosphoniumsalzen der Formel

$$(H_5C_6)_3\overset{+}{\underset{\underset{X}{-}}{P}}-CH_2-CH(OC_2H_5)_2 \qquad \text{(VIII)}$$

in welcher X für Halogen steht, mit einem Alkalialkoholat bei Temperaturen zwischen -50 und 100°C und anschließender Hydrolyse der dabei erhaltenen Acetale der Formel

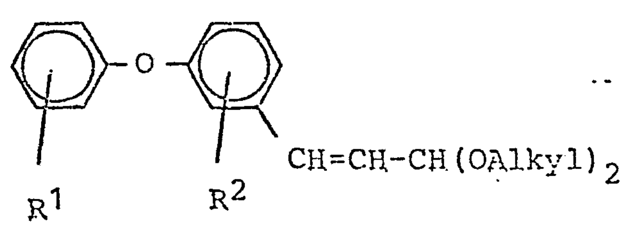

(IX)

mittels einer starken Säure, wie z.B. Salzsäure. Schließlich kann man sie auch durch Umsetzung von Verbindungen der Formel (VI), in welcher R für niederes Alkyl steht, mit einer Lösung des Vilsmeier-Haack-Reagenz Dimethylformamid-Phosphoroxichlorid in Dimethylformamid bei Temperaturen zwischen -50 und +100°C erhalten.

Ester der Formel (III) erhält man z.B., indem man Aldehyde der Formel (VI)

(a) mit Carbonsäureestern, z.B. Ethylacetat, und einem Alkalialkoholat bei Temperaturen zwischen 0 und 100°C oder

(b) mit Phosphonestern der Formel

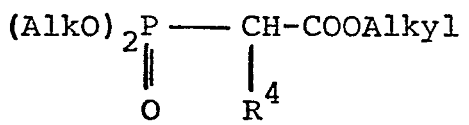 (X)

und einem Alkalialkoholat in einem niederen Alkohol oder mit Natriumhydrid in Dimethylformamid oder Dimethoxyethan umsetzt.

Die Säurechloride der Formel (IV) schließlich kann man erhalten, in dem man Aldehyde der Formel (VI)

(a) mit Malonsäure unter Wasser- und Kohlendioxidabspaltung in einem organischen Lösungsmittel und in Gegenwart eines Katalysators, z.B. Piperidin, bei Temperaturen zwischen 20 und 150°C oder

(b) mit Acetanhydrid und gegebenenfalls einem Alkaliacetat als Katalysator bei Temperaturen zwischen 80 und 200°C umsetzt

und die beim Ansäuern erhaltenen Säuren mit einem Halogenierungsmittel, z.B. Thionylchlorid, reagieren läßt.

Die Phenoxybenzaldehyde und -ketone der Formel (VI) sind bekannte Verbindungen (vgl. DE-OS 26 24 360, DE-OS 29 51 496, DE-OS 28 50 180, DE-OS 27 09 264, EP 3 427 EP 12 850) bzw. können nach bekannten Methoden synthetisiert werden /Nachrichten aus Chemie, Technik und Laboratorium 26, 120 (1078)/.

Die Ketoverbindungen der Formel (VII) sind gleichfalls bekannt, ebenso die Triphenylphosphoniumsalze der Formel (VIII) und die Phosphonester der Formel (X).

Die Verbindungen der Formel I sind wertvolle Ausgangsstoffe zur Herstellung von pestiziden Wirkstoffen. Beispielsweise kann man aus ihnen durch Umsetzung mit substituierten Cyclopropancarbonsäuren stark wirksame Insektizide vom Pyrethroid-typ erhalten.

Herstellungsbeispiele

Beispiel 1:

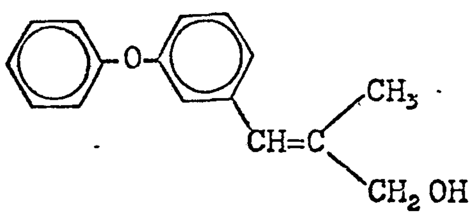

23,8 g (0,1 Mol) 2-Methyl-3-(3-phenoxyphenyl)-2-propenal werden in 100 ml Methanol/Ethanol-Gemisch gelöst und bei 0-10°C portionsweise während 30 Minuten mit 3,8 g (0,1 Mol) Natriumborhydrid versetzt. Anschließend wird zwei Stunden bei 25°C nachgerührt, mit 2n Schwefelsäure vorsichtig angesäuert und die Lösung im Vakuum eingeengt. Das verbleibende Gemisch wird 2x mit jeweils 100 ml Ether extrahiert, mit gesättigter Kochsalzlösung gewaschen und über Natriumsulfat getrocknet. Nach dem Abdestillieren des Ethers im Vakuum und Destillation erhält man 21,2 g (88 % der Theorie) 2-Methyl-3-(3-phenoxiphenyl)-2-propenol, Siedepunkt: 161 - 166°C/ 1.3 mbar, $n_D^{22}$=1,6015.

Beispiel 2:

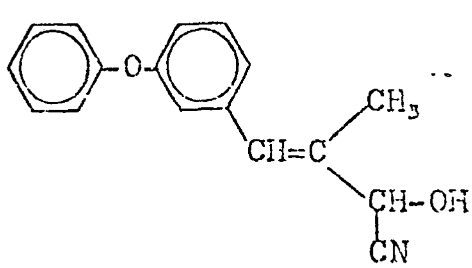

./.

23,8 g (0,1 Mol) 2-Methyl-3-(3-phenoxyphenyl)-2-propenal werden in 25 ml Eisessig gelöst und bei 15°C unter Rühren eine Lösung von 10,2 g Natriumcyanid in 25 ml $H_2O$ zugetropft. Anschließend rührt man 8 Stunden bei 20°C, gießt in 100 ml Wasser und extrahiert mit 200 ml Ether. Die Ether-Phase wird mit verdünnter Natriumbicarbonatlösung und gesättigter Kochsalzlösung gewaschen und über Natriumsulfat getrocknet. Nach dem Abdestillieren des Ethers im Vakuum erhält man 20 g (76 % der Theorie) 2-Hydroxy-3-methyl-4-(3-phenoxiphenyl)-3-butenonitril mit dem Brechungsindex $n_D^{21}=1,6010$.

Beispiel 3:

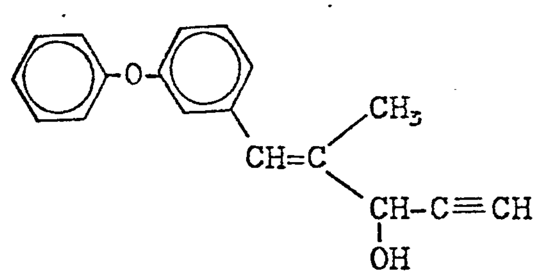

7,2 g Magnesiumspäne werden in 200 ml wasserfreiem Tetrahydrofuran bei 30 - 40°C mit 42 g (0,36 Mol) Ethylbromid versetzt und nach Ende der Zugabe 40 Minuten bei 50°C nachgerührt. Diese Lösung tropft man zu einer bei 20°C gesättigten Lösung von Acetylen in 100 ml Tetrahydrofuran, wobei weiter Acetylen eingeleitet wird. Nach beendetem Zutropfen wird noch eine Stunde Acetylen eingeleitet. Zu dieser Suspension

./.

werden bei 30°C 35,7 g (0,15 Mol) 2-Methyl-3-(3-phenoxyphenyl)-2-propenal in 50 ml wasserfreiem Tetrahydrofuran getropft und 4 Stunden bei 40°C gehalten. Danach kühlt man auf 10°C ab, gießt auf 1000 ml Eiswasser und löst den Niederschlag mit konz. Salzsäure auf. Man extrahiert 2x mit jeweils 100 ml Ether, schüttelt die Ether-Phase mit Wasser und gesättigter Kochsalzlösung und trocknet über Natriumsulfat. Nach Entfernen des Lösungsmittels im Vakuum erhält man 37,6 g (95 % der Theorie) 4-Methyl-5-(3-phenoxyphenyl)-4-penten-1-in-3-ol mit dem Brechungsindex $n_D^{26}=1,6040$.

Beispiel 4:

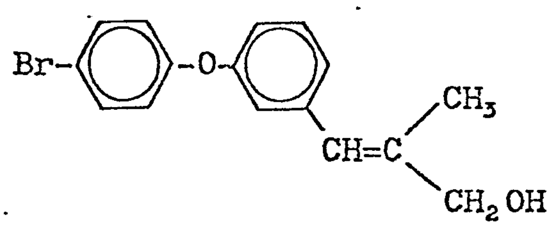

Zu 2,5 g Lithiumaluminiumhydrid in 50 ml wasserfreiem Tetrahydrofuran wird bei 25°C eine Lösung von 10,83 g (0,03 Mol) α-Methyl-3-(4-bromphenoxy)zimtsäureethylester in 50 ml Tetrahydrofuran getropft. Man rührt 20 Stunden bei 25°C, kühlt auf 0°C und tropft solange Eiswasser zu, bis keine Wasserstoffentwicklung mehr zu beobachten ist. Den Niederschlag löst man mit 30 ml konz. Salzsäure auf, extrahiert 2x mit jeweils 100 ml Toluol und trocknet die Toluol-Phase über Natriumsulfat. Nach Entfernen des Lösungsmittels im Vakuum

./.

erhält man 5,26 g (55 % der Theorie) 2-Methyl-3-[3-(4-bromphenoxy)phenyl]-2-propenol mit dem Brechungsindex $n_D^{21}=1{,}6243$.

Beispiel 5:

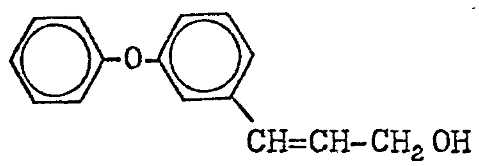

Zu 4,2 g Lithiumaluminiumhydrid in 200 ml wasserfreiem Ether tropft man bei 10°C eine Lösung von 51,7 g (0,2 Mol)3-Phenoxyzimtsäurechlorid in 50 ml Ether, rührt eine Stunde bei 10°C, sowie 4 Stunden bei 25°C. Man kühlt auf 0°C ab, versetzt vorsichtig mit Eiswasser, bis sich kein Wasserstoff mehr entwickelt, löst den Niederschlag mit konz. Salzsäure auf, extrahiert 2x mit je 200 ml Toluol und trocknet die organische Phase über Natriumsulfat. Nach Entfernen des Lösungsmittels im Vakuum erhält man 40,4 g (89 % der Theorie) 3-(3-Phenoxyphenyl)-2-propenol mit dem Brechungsindex $n_D^{23}=1{,}6144$.

Analog einem der Beispiele 1 bis 5 können die Verbindungen der Formel (I)

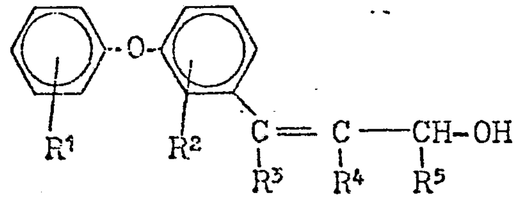 (I)

hergestellt werden! ./.

| Beispiel | $R^1$ | $R^2$ | $R^3$ | $R^4$ | $R^5$ | Brechungsindex |
|---|---|---|---|---|---|---|
| 6 | H | H | H | H | CN | $n_D^{23}$=1,6089 |
| 7 | H | H | H | H | C≡CH | $n_D^{24}$=1,5992 |
| 8 | H | H | H | H | $CH_3$ | $n_D^{26}$=1,5961 |
| 9 | 4-Br | H | H | H | H | $n_D^{23}$=1,6390 |
| 10 | 4-Br | H | H | H | CN | |
| 11 | 4-Br | H | H | H | C≡CH | |
| 12 | 4-Br | H | H | H | $CH_3$ | $n_D^{23}$=1,6207 |
| 13 | 4-Cl | H | H | H | H | $n_D^{23}$=1,6250 |
| 14 | 4-Cl | H | H | H | CN | |
| 15 | 4-Cl | H | H | H | C≡CH | |
| 16 | 4-F | H | H | H | H | $n_D^{24}$=1,6049 |
| 17 | 4-F | H | H | H | CN | |

./.

Fortsetzung Tabelle

| Beispiel | $R^1$ | $R^2$ | $R^3$ | $R^4$ | $R^5$ | Brechungs-index |
|---|---|---|---|---|---|---|
| 18 | 4-F | H | H | H | C≡CH | |
| 19 | $4-CH_3$ | H | H | H | H | $n_D^{23}=1,6101$ |
| 20 | $4-CH_3$ | H | H | H | CN | |
| 21 | $4-CH_3$ | H | H | H | C≡CH | |
| 22 | $4-OCH_3$ | H | H | H | H | $n_D^{22}=1,6118$ |
| 23 | $4-OCH_3$ | H | H | H | CN | |
| 24 | 3-Br | H | H | H | H | $n_D^{21}=1,6408$ |
| 25 | 3-Cl | H | H | H | H | $n_D^{21}=1,6298$ |
| 26 | $3-CH_3$ | H | H | H | H | |
| 27 | $2-CH_3$ | H | H | H | H | $n_D^{21}=1,6340$ |
| 28 | H | $4-C(CH_3)_3$ | H | H | H | $n_D^{25}=1,5943$ |

./.

Fortsetzung Tabelle

| Beispiel | $R^1$ | $R^2$ | $R^3$ | $R^4$ | $R^5$ | Brechungs-index |
|---|---|---|---|---|---|---|
| 29 | H | $4-C(CH_3)_3$ | H | H | CN | |
| 30 | H | $4-C(CH_3)_3$ | H | H | C≡CH | |
| 31 | H | 4-F | H | H | H | $n_D^{20}=1,6181$ |
| 32 | H | 4-F | H | H | CN | |
| 33 | H | 4-F | H | H | C≡CH | |
| 34 | 4-Cl | $4-C(CH_3)_3$ | H | H | H | |
| 35 | 4-Cl | $4-C(CH_3)_3$ | H | H | CN | |
| 36 | 4-Cl | 4-F | H | H | H | $n_D^{23}=1,6029$ |
| 37 | 4-Cl | 4-F | H | H | CN | |
| 38 | $4-CH_3$ | 4-F | H | H | H | $n_D^{25}=1,5895$ |

./.

Fortsetzung Tabelle

| Beispiel | $R^1$ | $R^2$ | $R^3$ | $R^4$ | $R^5$ | Brechungs-index |
|---|---|---|---|---|---|---|
| 39 | 4-$CH_3$ | 4-F | H | H | CN | |
| 40 | H | H | H | Br | H | $n_D^{22}=1,6270$ |
| 41 | H | H | H | Br | CN | |
| 42 | H | H | H | Br | $CH_3$ | $n_D^{23}=1,6121$ |
| 43 | 4-Br | H | H | Br | H | |
| 44 | 4-Br | H | H | Br | CN | |
| 45 | 4-Br | H | H | Br | $CH_3$ | |

./.

Fortsetzung Tabelle

| Beispiel | $R^1$ | $R^2$ | $R^3$ | $R^4$ | $R^5$ | Brechungs-index |
|---|---|---|---|---|---|---|
| 46 | 4-Br | H | H | $CH_3$ | CN | $n_D$ |
| 47 | 4-Br | H | H | $CH_3$ | C≡CH | |
| 48 | H | H | Br | $CH_3$ | H | $n_D^{20}=1,6120$ |
| 49 | H | H | Br | $CH_3$ | CN | $n_D$ |
| 50 | 4-Br | H | Br | $CH_3$ | H | $n_D$ |
| 51 | 4-Br | H | Br | $CH_3$ | CN | $n_D$ |
| 52 | H | H | H | $C_2H_5$ | H | $n_D^{27}=1,5894$ |
| 53 | H | H | H | $C_2H_5$ | CN | $n_D$ |
| 54 | H | H | H | $C_2H_5$ | C≡CH | |
| 55 | 4-Br | H | H | $C_2H_5$ | H | $n_D$ |
| 56 | 4-Br | H | H | $C_2H_5$ | CN | |

./.

Fortsetzung Tabelle

| Beispiel | $R^1$ | $R^2$ | $R^3$ | $R^4$ | $R^5$ | Brechungs-index |
|---|---|---|---|---|---|---|
| 57 | H | H | H | $C_6H_5$ | H | |
| 58 | H | H | H | $C_6H_5$ | CN | |
| 59 | H | H | H | 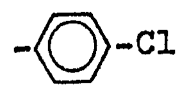 | H | |
| 60 | H | H | H | 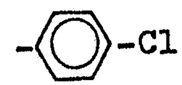 | CN | |
| 61 | H | H | Cl | H | H | $n_D$ |
| 62 | H | H | Cl | H | CN | |
| 63 | 4-Br | H | Cl | H | H | |
| 64 | 4-Br | H | Cl | H | CN | |
| 65 | H | $CF_3$ | H | H | H | |
| 66 | H | H | $CH_3$ | H | H | |
| 67 | H | H | CN | H | H | |
| 68 | H | H | H | Cl | H | |

./.

Herstellung der Ausgangsverbindungen:

Nach den in den Beispielen a - e dargelegten Methoden können die Ausgangsverbindungen der Formel II, III und IV hergestellt werden.

(a)

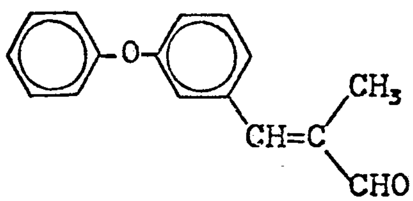

Zu einer Lösung von 39,6 g (0,2 Mol) 3-Phenoxybenzaldehyd und 17,4 g (0,3 Mol) Propionaldehyd in 300 ml Ethanol werden bei 0 - 10°C 60ml 3,6n Natronlauge zugetropft und 6 Stunden bei 25°C nachgerührt. Anschließend wird die Lösung mit konz. Schwefelsäure angesäuert und im Vakuum eingeengt. Der verbleibende Rückstand wird in 200 ml Toluol aufgenommen, mit Wasser und gesättigter Kochsalzlösung ausgeschüttelt und über Natriumsulfat getrocknet. Nach Entfernen des Lösungsmittels im Vakuum und Destillation erhält man 40,7 g (86 % der Theorie) 2-Methyl-3-(3-phenoxyphenyl)-2-propenal vom Siedepunkt Kp: 170-174°C/1 Torr; $n_D^{20}$=1,6273.

(b)

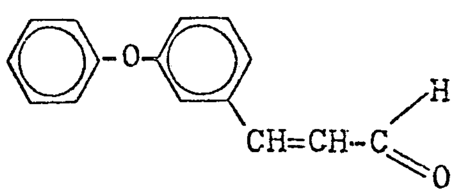

Eine Lösung von 2,3 g (0,1 Mol) Natrium in 50 ml Ethanol wird tropfenweise zu einer Lösung von 45,9 g (0,1 Mol)

./.

Diethoxyethyltriphenylphosphoniumbromid in 200 ml Ethanol bei 0°C gegeben. Danach tropft man 19,8 g (0,1 Mol) 3-Phenoxybenzaldehyd zu, beläßt 2 Stunden bei 0°C, sowie 2 Stunden bei 25°C. Nach dem Einengen wird mit Toluol extrahiert, filtriert, getrocknet und das Lösungsmittel im Vakuum abdestilliert. Man erhält 25,7 g (86 % der Theorie) 3-Phenoxyzimtaldehyddiethylacetal, welches mit 100 ml 4n HCl zwei Stunden auf 50°C erwärmt wird. Man extrahiert 2x mit jeweils 50 ml Ether, schüttelt die Ether-Phase mit gesättigter Kochsalzlösung und trocknet über Natriumsulfat. Nach Abdestillieren des Lösungsmittels im Vakuum und Destillation erhält man 14,5 g (65 % der Theorie) 3-(3-Phenoxiphenyl)-2-propenal, Kp: 150 - 160°C/0,3 Torr; $n_D^{25}$=1,6339.

(c) 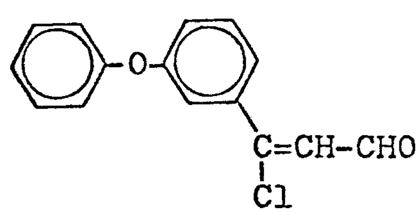

60 g Phosphoroxichlorid werden bei 5 - 10°C mit 50 ml Dimethylformamid versetzt und 30 Minuten nachgerührt. Innerhalb einer Stunde gibt man bei 10°C 42,4 g (0,2 Mol) 3-Phenoxyacetophenon hinzu und rührt nach Ende der Zugabe 4 Stunden bei 25°C. Die Lösung wird danach auf Eiswasser geschüttelt und mit 200 ml Dichlormethan extrahiert. Die organische Phase wird mit 10 %iger Sodalösung und Wasser ausgeschüttelt und über Natriumsulfat getrocknet. Nach Entfernen des Lösungsmittels im Vakuum erhält man 38,2 g (74 %) 3-Chlor-3-(3-phenoxiphenyl)-2-propenal, $n_D^{25}$=1,6509.

./.

(d) 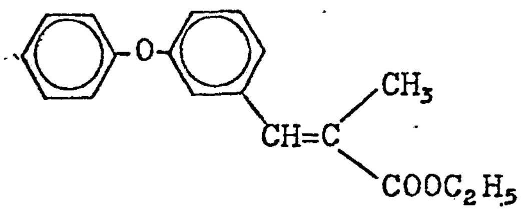

Eine Suspension aus 3 g 80 %igem Natriumhydrid in 60 ml Dimethoxyethan wird bei 30°C tropfenweise mit 23,8 g (0,1 Mol) (1-Ethoxycarbonylethyl)diethylphosphonat versetzt und 2 Stunden bei 30°C nachgerührt. Bei 15°C tropft man eine Lösung von 19,8 g (0,1 Mol) 3-Phenoxybenzaldehyd in 50 ml Dimethoxyethan zu und rührt 2 Stunden bei 15°C, gießt auf 200 ml Wasser und extrahiert 2x mit je 50 ml Ether; anschließend wird die Ether-Phase mit gesättigter Kochsalzlösung geschüttelt und über Natriumsulfat getrocknet. Nach dem Entfernen des Lösungsmittels im Vakuum erhält man 22,8 g (81 % der Theorie) 2-Methyl-3-(3-phenoxiphenyl)-2-propensäureethylester; Kp:105-109°C/0,8 Torr.

(e) 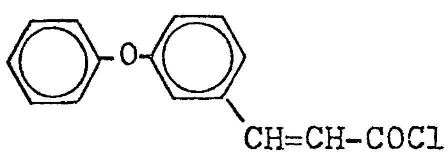

62,4 g Malonsäure werden in 100 ml Pyridin gelöst. Nach dem Abklingen der exothermen Reaktion gibt man 99 g 3-Phenoxybenzaldehyd und 5 g Piperidin hinzu, erhitzt 3 Stunden unter Rückfluß, kühlt auf 25°C ab und gießt auf Eis/konz. Salzsäure, um Pyridin und Piperidin herauszuwaschen. Die Säure scheidet sich dabei kristallin ab. Nach dem Trocknen erhält man

./.

113,7 g (95 % der Theorie) 3-Phenoxyzimtsäure, Fp: 107-109°C. Eine Suspension aus 24 g (0,1 Mol) 3-Phenoxyzimtsäure in 100 ml Toluol wird mit 18 g (0,15 Mol) Thionylchlorid versetzt und 3 Stunden bei 30°C gerührt, das Lösungsmittel im Vakuum entfernt und destilliert. Man erhält 24,6 g (95 % der Theorie) 3-(3-Phenoxiphenyl)-2-propensäurechlorid, Kp: 155-158°C/0,2 Torr.

Patentansprüche:

1. Phenoxyzimtalkohole der Formel

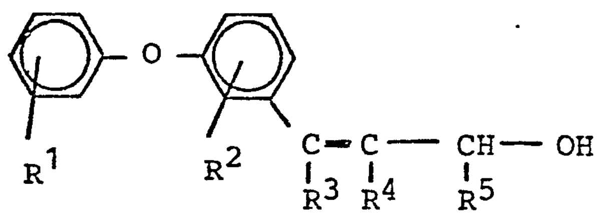

(I)

in welcher
$R^1$ Wasserstoff, Halogen, $(C_1-C_4)$Alkyl oder $(C_1-C_4)$Alkoxy,
$R^2$ Wasserstoff, Halogen, $(C_1-C_4)$Alkyl oder Trifluormethyl,
$R^3$ Wasserstoff, Cl, Br, $(C_1-C_4)$Alkyl oder Cyan
$R^4$ Wasserstoff, Halogen, $(C_1-C_4)$Alkyl, Phenyl oder Halogenphenyl,
$R^5$ Wasserstoff, $(C_1-C_4)$Alkyl, $(C_1-C_3)$Alkinyl oder Cyan
bedeuten.

2. Verbindungen der Formel I in Form ihrer optischen Enantiomeren bzw. ihrer Stereoisomeren.

3. Verfahren zur Herstellung von Verbindungen der Formel I, dadurch gekennzeichnet, daß man
(a) Verbindungen der Formel

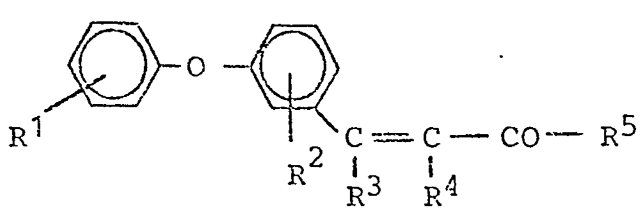

(II)

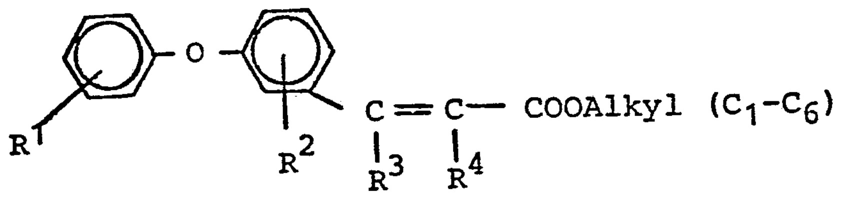

(III)

oder

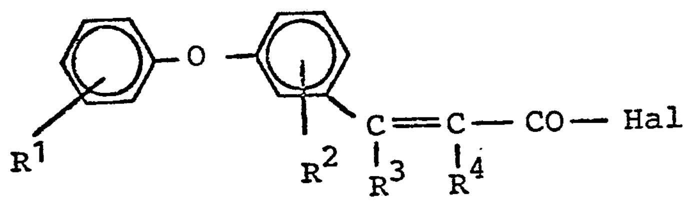

(IV)

reduziert, oder

(b) falls $R^5$ = CN,
Verbindungen der Formel (II) mit einem Alkali- oder Erdalkalicyanid in Gegenwart einer Säure bzw. mit Trimethylsilylcyanid behandelt, oder

(c) falls $R^5$ = Alkinyl,
Verbindungen der Formel (II) mit einer Grignard-Verbindung der Formel

$$R^6-C\equiv C-MgHal \qquad (V)$$

worin $R^6$ H oder $CH_3$ ist, umsetzt.

4. Verwendung von Verbindungen der Formel I als Ausgangsstoffe zur Herstellung von Insektiziden.

**0050857**

Europäisches Patentamt

# EUROPÄISCHER RECHERCHENBERICHT

Nummer der Anmeldung

EP 81108801.2

| EINSCHLÄGIGE DOKUMENTE | | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | |
| | DE - A1 - 2 502 786 (MERCK)<br>* Patentanspruch 1 *<br>-- | 1 | C 07 C 43/295<br>C 07 C 121/75 |
| | EP - A1 - 0 008 333 (CIBA-GEIGY AG)<br>* Patentansprüche 1,7,8 *<br>-- | 1,3,4 | |
| | AT - B - 358 022 (ROUSSEL-UCLAF)<br>* Seite 2, Zeilen 1-6; Seite 3, Zeilen 21-27 *<br>---- | 1,2,4 | |

RECHERCHIERTE SACHGEBIETE (Int. Cl.)

C 07 C 41/00
C 07 C 43/00
C 07 C 121/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| WIEN | 22-12-1981 | REIF |

EPA form 1503.1 06.78